Europäisches Patentamt

European Patent Office

Office européen des brevets

(19)

(11) Veröffentlichungsnummer: **0 216 016**
**A2**

(12) # EUROPÄISCHE PATENTANMELDUNG

(21) Anmeldenummer: 86107554.7

(22) Anmeldetag: 03.06.86

(51) Int. Cl.⁴: **A 61 L 27/00**

(30) Priorität: 24.06.85 DD 277666

(43) Veröffentlichungstag der Anmeldung:
**01.04.87 Patentblatt 87/14**

(84) Benannte Vertragsstaaten:
**AT CH DE FR GB IT LI SE**

(71) Anmelder: Friedrich-Schiller-Universität Jena
Goetheallee 1
DDR-6900 Jena(DD)

(72) Erfinder: Vogel, Werner, Prof. Dr.
Schillbachstrasse 11
DDR-6900 Jena(DD)

(72) Erfinder: Heublein, Günther, Prof. Dr.
Friedensstrasse 30
DDR-6900 Jena(DD)

(72) Erfinder: Höland, Wolfram, Dr. rer. nat.
Judith-Auer-Strasse 9
DDR-6902 Jena(DD)

(72) Erfinder: Böse, Manfred
Döbereinerstrasse 50
DDR-6900 Jena(DD)

(72) Erfinder: Naumann, Karin, Dr.
Griesbachstrasse 7
DDR-6900 Jena(DD)

(72) Erfinder: Carl, Gunter, Dr.
W.-Pieck-Strasse 26
DDR-6902 Jena(DD)

(72) Erfinder: Vogel, Jürgen
S.-Allende-Platz 23
DDR-6902 Jena(DD)

(72) Erfinder: Wange, Peter
M.-Gorki-Strasse 3
DDR-6900 Jena(DD)

(72) Erfinder: Gummel, Jens, Dr.

DDR-1140 Berlin(DD)

(72) Erfinder: Zinner, Peter, Prof. Dr.
W.-Pieck-Strasse 36
DDR-6902 Jena(DD)

(72) Erfinder: Beleites, Eggert, Dr.

DDR-6901 Maua, Nr. 41(DD)

(72) Erfinder: Schubert, Thomas, Dr.
Hoyerswerdaer Strasse 29
DDR-8060 Dresden(DE)

(74) Vertreter: Zipse + Habersack
Kemnatenstrasse 49
D-8000 München 19(DE)

(54) **Anorganisch-organische Verbundwerkstoffe für biomedizinische Zwecke.**

(57) Die Erfindung betrifft anorganisch-organische Verbundwerkstoffe für biomedizinische Zwecke. Die Aufgabe der Erfindung besteht darin, anorganisch-organische Verbundwerkstoffe zu entwickeln, die für biomedizinische Zwecke in sehr breitem Umfang gezielt einstellbare Eigenschaften besitzen. Die Aufgabe wird dadurch gelöst, daß anorganische Ausgangsstoffe mit organischen Ausgangsstoffen eine neue feste chemische Verbindung darstellen, wobei die anorganische Ausgangskomponente ein biokompatibles Silikatglas und/oder eine Silikatglaskeramik des Systems $SiO_2$-$Al_2O_3$-MgO-$Na_2O$/$K_2O$-F und/oder ein bioaktives Phosphosilikatglas und/oder eine Phospholikatglaskeramik des Systems $SiO_2$-$Al_2O_3$-MgO-$Na_2O$/ $K_2O$-CaO-$P_2O_5$F und/oder des Systems $SiO_2$-MgO-$K_2O$-F -CaO-$P_2O_5$ darstellt und/oder die anorganische Ausgangskomponente ein bioaktives Phosphatglass und/oder Phosphatglaskeramik des Systems $P_2O_5$-$Al_2O_3$-CaO-$Na_2O$/ $K_2O$ und/oder eine auf der Basis von Tricalziumphosphat und/oder Apatit beruhende Sinterkeramik darstellt und die organische Ausgangskomponente ein biokompatibler, monomerfreier, nur aus den Elementen C, H, O zusammengesetzter epoxydierter polymerer Kohlenwasserstoff mit einer mittleren Molmasse M von 2 000 bis 6 000 und Epoxidäquivalenten EÄG von 50 bis 500 g darstellt und die anorganische Ausgangskomponente ein phosphorsäuremodifizierter Stoff darstellt.

Anorganisch-organische Verbundwerkstoffe für biomedizinische Zwecke

Die Erfindung betrifft anorganisch-organische Verbundwerkstoffe für biomedizinische Zwecke, die z. B. in Form von modellierbarem Hartgewebeersatz oder als Implantatmaterial mit unterschiedlichen Eigenschaften angewendet werden können.

In der Humanmedizin kommen in breitem Umfang, z. B. für das Einbetten von Endoprothesen oder als polymerisierende Zahnfüllmassen Materialien zum Einsatz, die niedermolekulare organische Verbindungen darstellen und nach Einbringen in den menschlichen Körper zu festen Produkten polymerisieren. Oftmals werden den organischen Stoffen auch anorganische Zusatzkomponenten hinzugefügt, so daß Kompositwerkstoffe entstehen, die jedoch untereinander keine chemische Bindung eingehen. Diese Materialien besitzen den Nachteil, daß in den ersten Minuten nach Einbringen in den Körper nichtreagierte Monomere mit dem Blut abtransportiert werden können und sich in bestimmten Körperorganen anreichern. Weiterhin bleiben im Prozeß der Polymerisation zwangsläufig nichtreagierte Monomere bzw. Oligomere im ausgehärteten Material erhalten und verbleiben somit im Körper, denn in der organischen Chemie durchgeführte Reinigungsoperationen, wie vielstufige Extraktions- und Fällungsreaktionen, können nicht angewendet werden. So treten z. B. nach Schultz, Arch. Orthop. Unfall-Chir., 21 1971, 301 - 315 bei der Verwendung von Knochenzement auf der Basis von Methylmethacrylat neben Blutdruckabfall vor allem lebensbedrohliche Sofortkomplikationen durch Fettembolien auf. Weiterhin werden nach T. Okano, Polym. Journal, 10, 233 (1978), an den hydrophilen Struktureinheiten des gehärteten Materials Proteine adsorbiert, was nach M. Suzuki, J. Biomed. Mat. Res. 15, 697 (1981) bis zur Bildung von Blutgerinsel führen kann. Generell sind ähnliche Reaktionen, die körperschädigende Prozesse bewirken und damit die geforderte hohe Biokompatibilität nicht gewährleisten, von allen organischen bzw. anorganisch-organischen Werkstoffen zu erwarten, die von niedermolekularen Verbindungen ausgehen und/oder aus anderen als den Elementen C, H, O bestehen. Das trifft z. B. für die DE-OS 2.821.354 zu. Z. B. können bei einigen in der vorgenannten Offenlegungsschrift erwähnten Polymeren, wie etwa Polyurethanen, Polyamiden oder Epoxyharzen als Biomaterial schädigende Einflüsse auf das biologische Medium durch nichtreagierte Isocyanatgruppen oder gebildete Oligomere entstehen.

Neben den möglichen Reaktionen von Isocyanatgruppen mit der Körperflüssigkeit, dem Körpergewebe, Blutkörperchen oder einzelnen Proteinen entstehen aus den Polyurethanen durch Hydrolyse Aminogruppen enthaltende Metaboliten, die z. B. im Fall der aromatischen Diamine potentielle Carzinogene darstellen. Auch die Oligomeren sind nach N. A. Mohamud, Physicochem. Aspects of Polymer Surfances, Vol. 2, S. 953, Plenum Press, New York, London, 1983, in der Lage, langsam an die Oberfläche des Materials zu diffundieren und unerwünschte Blutreaktionen hervorzurufen. Für extrakorporale Applikationen, aber auch für Implantate vorgesehenes Containermaterial auf Polyurethanbasis, werden aufwendige Extraktionsmethoden sowie Oberflächenmodifizierungen des Materials angewendet. Mit steigender Viskosität im Härtungsprozeß werden naturgemäß infolge der gebundenen Diffusion bei Makromolekülen nichtreagierte funktionelle Gruppen verbleiben, die mit der Zeit durch eindiffundierendes Wasser oder Ionen aus dem Liquor reagieren und niedermolekulare Stoffe mit Amid- bzw. Aminbindungen freisetzen. Abgesehen von ihrer möglichen Toxizität wird besonders durch die starke Basizität von Aminen und Diaminen die Blutgerinnung stark gefördert. Aus dem gleichen Grunde sollten Amine als Härtungskomponenten, z. B. für Epoxydharze nicht verwendet werden, da der in Langzeitanwendungen nicht auszuschließende hydrolytische bzw. enzymatische Abbau solcher Verbunde potentielle Gefahren impliziert.

Die Aufgabe der Erfindung besteht darin, anorganisch-organische Verbundstoffe zu entwickeln, die für biomedizinische Zwecke in sehr breitem Umfang gezielt einstellbare Eigenschaften besitzen.

Diese Aufgabe wird erfindungsgemäß dadurch gelöst, daß anorganische Ausgangstoffe mit organischen Ausgangsstoffen eine neue feste chemische Verbindung darstellen, wobei die anorganische Ausgangskomponente ein biokompatibles Silikatglas und/oder eine Silikatglaskeramik der Zusammensetzung in Masse-%

$SiO_2$ = 34 - 60
$Al_2O_3$ = 21 - 36
$MgO$ = 8 - 17
$R_2O$ = 5 - 12
$F$ = 1 - 7

wobei $R_2O$ die Summe von 0 - 8 Ma.-% $Na_2O$ und 0 - 6 Ma.-% $K_2O$ darstellt,

und/oder die anorganische Ausgangskomponente ein bioaktives Phosphosilikatglas und/oder eine Phosphosilikatglaskeramik (A) der Zusammensetzung in Masse-%

$SiO_2$ = 19 - 52
$Al_2O_3$ = 12 - 23
$MgO$ = 5 - 15
$R_2O$ = 3 - 10
$CaO$ = 9 - 30
$P_2O_5$ = 4 - 24
$F^-$ = 0,5 - 7

wobei $R_2O$ die Summe von 0 - 8 Ma.-% $Na_2O$ und 0 - 8 Ma.-% $K_2O$ darstellt,

und/oder er die anorganische Ausgangskomponente ein bioaktives Phosphosilikatglas und/oder eine Phosphosilikatglaskeramik (B) der Zusammensetzung in Masse-%

$SiO_2$ = 18 - 63
$MgO$ = 10 - 35
$K_2O$ = 3 - 10
$F^-$ = 4 - 9
$CaO$ = 1 - 30
$P_2O_5$ = 1 - 30

darstellt,
und/oder die anorganische Ausgangskomponente ein bioaktives Phosphatglas und/oder eine Phosphatglaskeramik der Zusammensetzung in Masse-%

$P_2O_5$ = 43 - 58
$Al_2O_3$ = 3 - 21
$CaO$ = 8 - 26
$R_2O$ = 10 - 25

darstellt,

wobei $R_2O$ bis 25 Ma.-% $Na_2O$ und bis 18 Ma.-% $K_2O$ enthalten kann und/oder die anorganische Ausgangskomponente eine auf der Basis von Tricalziumphosphat und/oder Apatit beruhende Sinterkeramik darstellt und die organische Ausgangskomponente ein biokompatibler, monomerfreier, nur aus den Elementen C, H, O zusammengesetzter epoxydierter polymerer Kohlenwasserstoff mit einer mittleren Molmasse $\overline{Mn}$ von 2 000 bis 6 000 und Epoxidäquivalenten EAG von 50 bis 500 g darstellt und die anorganische Ausgangskomponente einen phosphorsäuremodifizierten Stoff darstellt. Weiterhin ist es möglich, daß die anorganische Ausgangskomponente in Form des Silikatglases und/oder der Silikatglaskeramik Zusatzstoffe bis 10 Masse-%, wie z. B. FeO, $Fe_2O_3$, CaO, $P_2O_5$, BaO, enthalten kann und die anorganische Ausgangskomponente in Form des bioaktiven Phosphosilikatglases und/oder der Phosphosilikatglaskeramik (A) Zusatzstoffe bis 10 Masse-%, wie z. B. FeO, $Fe_2O_3$, BaO, $TiO_2$, enthalten kann

und die anorganische Ausgangskomponente in Form des bioaktiven Phosphosilikatglases und/oder der Phosphosilikatglaskeramik (B) Zusatzstoffe bis 6 Ma.-%, wie z. B. $Al_2O_3$, $TiO_2$, $ZrO_2$, $Na_2O$, enthalten kann
und die anorganische Ausgangskomponente in Form des bioaktiven Phosphatglases und/oder der Phosphatglaskeramik Zusatzstoffe bis 10 Ma.-%, wie z. B. $TiO_2$, $B_2O_3$, $F^-$, $SiO_2$, FeO, $Fe_2O_3$, MgO, enthalten kann.

Die organische Ausgangskomponente ist erfindungsgemäß ein monomerfreier, nur aus den Elementen C, H, O bestehender epoxydierter polymerer Kohlenwasserstoff mit einer Molmasse $\overline{Mn}$ von 2 000 bis 6 000 und Epoxidäquivalenten EAG von 100 bis 500 g. Als Zusatzstoffe zur organischen Ausgangskomponente eignen sich z. B. Färbungskomponenten und/oder Antibiotika und/oder Komponenten, die zur Erhöhung der Hydrophilie beitragen, wie z. B. Poly (Vinylalkohol - Acrylsäure) - Copolymere.

Die erfindungsgemäßen Verbundwerkstoffe bestehen aus 20 - 70 Masse-% einer organischen Ausgangskomponente und 30 - 80 Masse-% einer anorganischen Ausgangskomponente, wobei durch die erfindungsgemäße 1 - 24-stündige Behandlung der anorganischen Komponente mit 30 - 50 %iger $H_3PO_4$, bei 25 bis 130 $^o$C und Vermischen beider Ausgangsstoffe Verbundwerkstoffe entwickelt werden, die sehr unterschiedliche Eigenschaften besitzen, nämlich z. B. von knorpelähnlichen bis knochenähnlichen Eigenschaften reichen. Bei der Bildung dieser erfindungsgemäßen Verbundwerkstoffe, die durch eine feste chemische Verbindung zwischen anorganischer und organischer Komponente charakterisiert sind, treten keinesfalls Temperaturen über 45 $^o$C auf. Damit ist die Anwendung als modellierbarer Hartgewebeersatz z. B. zum Einbetten von Endoprothesen, oder der Substitution von Knochendefekten oder als Knorpelersatzmaterial möglich.

Überraschend ist auch, daß erfindungsgemäß Implantate mit Langzeitstabilität und unterschiedlichen mechanischen Parametern, wie z. B. Festigkeit und E-Modul, die z. B. von knorpelähnlich bis knochenähnlich gezielt eingestellt werden können, entwickelt werden können. Erfindungsgemäß ist hierbei durch die Herstellung außerhalb des biologischen Mediums die Reaktionszeit, durch die Erhöhung der Reaktionstemperatur auf beispielsweise 100 $^o$C, zu kürzen. Aufgrund der einstellbaren Eigenschaften, der sehr guten Biokompatibilität und Bioaktivität können Implantate für nahezu alle Körperpartien des Menschen erzeugt werden.

Erfindungsgemäß ist eine schnelle, in ca. 20 Minuten erzielte 80 %ige Aushärtung durch 12-stündige Behandlung der anorganischen Ausgangskomponente mit 50 %iger $H_3PO_4$ bei 110 °C und Reaktionen mit der organischen Komponente zu erzielen, so daß die erfindungsgemäßen Verbundwerkstoffe z.B. als Zahnfüllmassen oder Dentalprodukte, wie z. B. Zahnaufbauten, auch in Kombination mit hochfesten Grundkörpern verwendet werden können.

Besonders vorteilhafte bioaktive Eigenschaften besitzen beispielsweise solche erfindungsgemäßen Verbundwerkstoffe, die als anorganische Ausgangskomponenten bioaktive Glaskeramiken, mit den Hauptkristallphasen Glimmer und Apatit, enthalten. Der Grund dafür ist, daß die anorganische Komponente eine direkte Bindung zum lebenden Knochen bei einer nur minimalen Reaktionszone von 5 bis 12 μm ausbildet. Diese Materialeigenschaft ist für die Anwendung des erfindungsgemäßen Verbundwerkstoffes als modellierbarer Hartgewebeersatz von entscheidender Bedeutung, denn die optimale Korngröße der anorganischen Ausgangskomponente für diesen Einsatzzweck beträgt 40 bis 460 μm. Bei anderen bekannten bioaktiven Glaskeramiken treten Reaktionszonen in der Glaskeramikoberfläche von mehr als 150 μm auf, so daß es zu einer beträchtlichen Festigkeitsminderung des Verbundes Glaskeramik - Knochen und vor allem zum Auflösen des Materials in einer Schichtdicke von mehr als 150 μm kommt, d. h., daß Partikel kleiner 150 μm aufgelöst werden.

Da die Reaktionszone zwischen der anorganischen Komponente

der erfindungsgemäßen Verbundwerkstoffe und dem lebenden Knochen nur 5 bis 12 μm beträgt, bleiben die Partikel erhalten und vor allem von den Apatitkristallen geht die bioaktive Eigenschaft zur Verbundbildung mit dem lebenden Knochen aus. Zusammenfassend bedeutet das, daß die anorganische und die organische Komponente durch Ausbildung einer direkten chemischen Bindung die erfindungsgemäßen Verbundwerkstoffe entstehen und daß ausgehend von der anorganischen Komponente die Bindung zum Knochen erzielt wird.

1. Das anorganische Ausgangsmaterial, das z. B. eine Glaskeramik mit den Hauptkristallphasen Apatit und Glimmer darstellt, wird mit 50 %iger Phosphorsäure bei 110 °C behandelt und die überschüssige Flüssigkeit in geeigneter Form abgetrennt. 55 Masseteile des so erhaltenen behandelten anorganischen Ausgangsmaterials werden mit 45 Masseteilen eines epoxidierten polymeren Kohlenwasserstoffs mit einer mittleren Molmasse von $\overline{M}n$ 3 700 und einen Epoxidäquivalentgewicht EÄG von 156 g intensiv vermischt. Nach 15 Minuten ist die Masse ausgehärtet und erreicht 80 % ihrer Festigkeit. Nach 24 h hat der erfindungsgemäße Verbundwerkstoff seine Endfestigkeit erreicht.

2. Das anorganische Ausgangsmaterial, daß z. B. eine Glaskeramik darstellt, die Glimmer als Hauptkristallphase enthält, wird mit 30 %iger Phosphorsäure 12 h bei 25 °C behandelt, die überschüssige Flüssigkeit entfernt und mit der organischen Komponente im Verhältnis von einem Teil anorganischer zu 2 Teilen organischer Komponente vermischt. Diese Masse ist noch 30 Minuten nach dem Vermischen modellierbar und hat nach 2 Stunden druckelasti-

sche Knorpelähnliche Eigenschaften und Festigkeitswerte. Der epoxidierte Kohlenwasserstoff hat eine mittlere Molmasse $\overline{M}n$ von 3 700 und ein Epoxidäquivalentgewicht EÄG von 250 g.

3. Das anorganische Ausgangsmaterial, das z. B. eine Glimmerglaskeramik darstellt, wird mit 50 %iger Phosphorsäure 12 h bei 110 °C behandelt und die überschüssige Flüssigkeit entfernt. Das so behandelte anorganische Ausgangsmaterial wird zu gleichen Teilen mit einem epoxidierten polymeren Kohlenwasserstoff, welcher eine mittlere Molmasse $\overline{M}n$ von 5 600 und ein Epoxidäquivalentgewicht EÄG von 110 g aufweist, vermischt. Der erfindungsgemäße Verbundwerkstoff ist nach 20 Minuten ausgehärtet und erreicht zu diesem Zeitpunkt 80 % seiner Endfestigkeit, die nach 24 h erreicht ist.

4. 60 Masseteile der nach Beispiel 1 behandelten anorganischen Komponenten werden innig vermischt und in einer geeigneten Form nach dem Entgasen bei 80 °C in 3 Stunden ausgehärtet. Die so gewonnenen Prüfkörper der Größe 2 x 2 x 2 mm bzw. 5 x 5 x 35 mm wurden entsprechend der Verfahrensweise bei den nach Beispiel 1 - 3 erhaltenen Reaktionskompositen auf ihre mechanische Festigkeit sowie ihre toxikologische Unbedenklichkeit hin überprüft. Dabei konnten Biegebruchfestigkeiten bis 180 MPa und E-Moduli bis $2,3 \cdot 10^4$ MPa erreicht werden. Sowohl die Ausgangskomponenten als auch die Probekörper, z. B. in zerkleinerter Form, zeigten im Vergleich zu anerkannten biokompatiblen Standarden, z. B. auf Zellkulturen, keine Nachteile. Die Auswertung teilexperimenteller Versuche ergab einen bindegewebsfreien Verbund der erfindungsgemäßen Verbundwerkstoffe mit bioaktiven Komponenten.

Tabelle 1 gibt Zusammensetzungen in Ma.-% und Hauptkristallphasen der anorganischen Ausgangskomponente
der erfindungsgemäßen Verbundwerkstoffe wieder.

Tabelle 1:

| | 1 | 2 | 3 | 4 | 5 | 6 |
|---|---|---|---|---|---|---|
| $SiO_2$ | – | – | – | 39,4 | 48,4 | 36,0 |
| $Al_2O_3$ | 20,9 | 12,9 | 8,8 | – | 29,1 | 20,0 |
| $MgO$ | – | – | – | 25,5 | 12,6 | 10,9 |
| $Na_2O$ | 15,9 | 16,8 | 14,3 | – | 3,2 | 2,3 |
| $K_2O$ | – | – | – | 6,9 | 4,3 | 3,9 |
| $CaO$ | 14,0 | 14,1 | 15,6 | 9,9 | – | 12,5 |
| $P_2O_5$ | 49,2 | 49,6 | 50,2 | 10,1 | – | 9,5 |
| $F^-$ | – | 1,7 | 1,8 | 6,4 | 2,7 | 4,9 |
| $FeO$ | – | 4,5 | 3,7 | – | – | – |
| $Fe_2O_3$ | – | 0,4 | – | – | – | – |
| $TiO_2$ | – | – | 5,6 | 2,0 | – | – |

Hauptkristallphase

| | | | | | |
|---|---|---|---|---|---|
| OH Apatit | | F-Apatit | | Glimmer | Glimmer |
| $AlPO_4$ | | $AlPO_4$ | | | Apatit |
| Phosphat-misch-phase | | (Berlinit) Phosphat-mischphase | | | |

F-Apatit
Phase vom
Berlinityp
Phosphat-
mischphase

Glimmer
Apatit

Patentansprüche:

1. Anorganisch-organische Verbundwerkstoffe für biomedizinische Zwecke, gekennzeichnet dadurch, daß anorganische Ausgangsstoffe mit organischen Ausgangsstoffen eine neue feste chemische Verbindung darstellen, wobei die anorganische Ausgangskomponente ein biokompatibles Silikatglas und/oder eine Silikatglaskeramik der Zusammensetzung in Masse-%.

$$
\begin{aligned}
SiO_2 &= 34 - 60 \\
Al_2O_3 &= 21 - 36 \\
MgO &= 8 - 17 \\
R_2O &= 5 - 12 \\
F &= 1 - 7
\end{aligned}
$$

wobei $R_2O$ die Summe von 0 - 8 Ma.-% $Na_2O$ und 0 - 6 Ma.-% $K_2O$ darstellt,
und/oder die anorganische Ausgangskomponente ein bioaktives Phosphosilikatglas und/oder eine Phosphosilikatglaskeramik (A) der Zusammensetzung in Masse-%

$$
\begin{aligned}
SiO_2 &= 19 - 52 \\
Al_2O_3 &= 12 - 23 \\
MgO &= 5 - 15 \\
R_2O &= 3 - 10 \\
CaO &= 9 - 30 \\
P_2O_5 &= 4 - 24 \\
F &= 0,5 - 7
\end{aligned}
$$

wobei $R_2O$ die Summe von 0 - 8 Ma.-% $Na_2O$ und 0 - 8 Ma.-% $K_2O$ darstellt,

und/oder die anorganische Ausgangskomponente ein bioaktives Phosphosilikatglas und/oder eine Phosphosilikatglaskeramik (B) der Zusammensetzung in Masse-%

$SiO_2$ = 18 - 63
MgO = 10 - 35
$K_2O$ = 3 - 10
$F^-$ = 4 - 9
CaO = 1 - 30
$P_2O_5$ = 1 - 30

darstellt,
und/oder die anorganische Ausgangskomponente ein bioaktives Phosphatglas und/oder eine Phosphatglaskeramik
der Zusammensetzung in Masse-%

$P_2O_5$ = 43 - 58
$Al_2O_3$ = 3 - 21
CaO = 8 - 26
$R_2O$ = 10 - 25

darstellt, wobei $R_2O$ bis 25 Ma.-% $Na_2O$ und bis 18 Ma.-%
$K_2O$ enthalten kann
und/oder die anorganische Ausgangskomponente eine auf
der Basis von Tricalziumphosphat und/oder Apatit beruhende Sinterkeramik darstellt und die organische Ausgangskomponente ein biokompatibler, monomerfreier, nur
aus den Elementen C, H, O zusammengesetzter epoxydierter
polymerer Kohlenwasserstoff mit einer mittleren Molmasse
$\bar{M}n$ von 2 000 bis 6 000 und Epoxidäquivalenten EAG von
50 bis 500 g darstellt und die anorganische Ausgangskomponente einen phosphorsäuremodifizierten Stoff darstellt.

2. Anorganisch-organische Verbundwerkstoffe nach Anspruch 1, dadurch gekennzeichnet, daß die anorganische Ausgangskomponente in Form des Silikatglases und/oder der Silikatglaskeramik Zusatzstoffe bis 10 Masse-% wie z. B. FeO, $Fe_2O_3$, CaO, $P_2O_5$, BaO, enthalten kann und die anorganische Ausgangskomponente in Form des bioaktiven Phosphosilikatglases und/oder der Phosphatsilikatglaskeramik (A) Zusatzstoffe bis 10 Masse-%, wie z. B. FeO, $Fe_2O_3$, BaO, $TiO_2$, enthalten kann und die anorganische Ausgangskomponente in Form des bioaktiven Phosphosilikatglases und/oder der Phosphosilikatglaskeramik (B) Zusatzstoffe bis 6 Ma.-%, wie z. B. $Al_2O_3$, $TiO_2$, $ZrO_2$, $Na_2O$, enthalten kann und die anorganische Ausgangskomponente in Form des bioaktiven Phosphatglases und/oder der Phosphatglaskeramik Zusatzstoffe bis 10 Ma.-%, wie z. B. $TiO_2$, $B_2O_3$, $F^-$, $SiO_2$, FeO, $Fe_2O_3$, MgO, enthalten kann.

3. Anorganisch-organische Verbundwerkstoffe nach Anspruch 1 und 2, dadurch gekennzeichnet, daß die Glaskeramik als anorganische Ausgangskomponente Glimmer und/oder Apatit, oder Apatit und $SiO_2$-isotype Kristalle als Hauptkristallphasen enthält.

4. Anorganisch-organischer Verbundwerkstoff nach Anspruch 1, dadurch gekennzeichnet, daß die organische Ausgangskomponente Zusatzstoffe, wie beispielsweise Färbungskomponenten und/oder Antibiotika und/oder Komponenten zur Erhöhung der Hydrophilie, wie z. B. Poly (Vinylalkohol - Acrylsäure) - Copolymere enthält.

5. Anorganisch-organische Verbundwerkstoffe nach Anspruch 1 bis 4, dadurch gekennzeichnet, daß die organische Ausgangskomponente 20 bis 70 Ma.-% darstellt und die anorganische Ausgangskomponente 30 - 80 Ma.-% darstellt.

6. Anorganisch-organische Verbundwerkstoffe nach Anspruch 1 bis 5, dadurch gekennzeichnet, daß die anorganische Ausgangskomponente mit 30 - 50 %iger $H_3PO_4$ 1 bis 24 h bei 25 bis 130 $^o$C behandelt wird und nach dem Vermischen mit der organischen Komponente zur Aushärtung kommt, wobei keinesfalls Temperaturen von 45 $^o$C überschritten werden, womit knorpelähnliche bis knochenähnliche Verbundwerkstoffe erzielt werden, die z. B. von Endoprothesen, Substitution von Knochendefekten oder als Substitutionsmaterial von knorpelähnlichen Körperpartien geeignet sind.

7. Anorganisch-organische Verbundwerkstoffe nach Anspruch 1 bis 5, dadurch gekennzeichnet, daß die anorganische Ausgangskomponente mit 30 - 50 %iger $H_3PO_4$ 1 - 24 h bei 25 - 130 $^o$C behandelt wird, so daß nach dem Vermischen mit der organischen Komponente und Aushärten in geeigneten Formen bei 25 bis 100 $^o$C Implantatwerkstoffe mit Langzeitstabilität, unterschiedlichen Eigenschaften, wie z. B. unterschiedlichen E-Moduli entstehen, die bioaktiv und/oder biokompatibel sind.

8. Anorganisch-organische Verbundwerkstoffe nach Anspruch 1 bis 5, dadurch gekennzeichnet, daß die anorganische Ausgangskomponente mit 50 %iger $H_3PO_4$ 12 h bei 110 $^o$C behandelt wird, so daß nach dem Vermischen mit der organischen Komponente innerhalb von 20 Minuten eine 80 %ige Aushärtung erreicht wird und ein fester Verbund, als z. B. Zahnfüllmasse oder Dentalprodukt in Form eines Zahnaufbaus mit dem Zahn und/oder dem Zahn und gleichzeitig einen hochfesten Grundkörper erzielt wird.